# EUROPEAN PATENT APPLICATION

(11) **EP 4 474 469 A2**
(43) Date of publication of application: **11.12.2024**
(21) Application number: 24179820.6
(22) Date of filing: 04.06.2024
(51) Int. Cl.: C12M 1/00, C12M 1/21

(54) **BUBBLE SEPARATING APPARATUS FOR SPECTROMETER AND BIOREACTOR INCLUDING THE SAME**

(30) Priority: 05.06.2023 KR 20230072362
(71) Applicant: Cubick, Seoul 03911 (KR)
(72) Inventor: Kim, Dongchoul, 04346 Yongsan-gu, Seoul (KR); Kang, Taewook, 07977 Yangcheon-gu, Seoul (KR); Lim, Youngwook, 07202 Yeongdeugpo-gu, Seoul (KR); Min, Junwon, 05812 Songpa-gu, Seoul (KR)
(74) Representative: Witte, Weller & Partner Patentanwälte mbB

(57) **Abstract**

Disclosed are a bubble separating apparatus for a spectrometer and a bioreactor including the same. According to an exemplary embodiment of the present disclosure, a bubble separating apparatus for a spectrometer detachable to a spectrometer, which irradiates light through an irradiation unit provided at one end, and analyzes an analysis object based on characteristics of the light transmitting the analysis object in a detection region formed at a front side of the irradiation unit may be provided, which includes: a housing including an opening provided so that at least a part of the spectrometer is inserted at one end, and an irradiation hole provided so that the light passes at the other end; and a flange formed at the other end of the housing to be longer than the detection region in an irradiation direction of the light.

## Description

### BACKGROUND

### Field

The present disclosure relates to a bubble separating apparatus for a spectrometer and a bioreactor including the same, and more particularly, to a bubble separating apparatus for a spectrometer and a bioreactor including the same which can prevent optical signal interference and a concentration measurement error by bubbles when analyzing a concentration, etc. from an analysis object in a bioreactor using a spectrometer.

### Description of the Related Art

In general, a bioreactor is widely used in a biotechnology industry, as biological reactions or processes can be carried out on a laboratory or industrial scale.

Products that can be produced through such a bioreactor can include various materials derived from foods, beverages, pharmaceuticals, bio-based chemicals, plastic, biofuels and cell culture.

On the other hand, when the cell culture is grown using such a bioreactor, a monitoring method of the cell culture generally collects and analyzes a sample from the bioreactor every time of a predetermined interval. However, such a conventional monitoring method has a problem in that the sample collected to the outside from the bioreactor is contaminated or the rapid change generated in the cell culture is not detected during the time when the monitoring is not made.

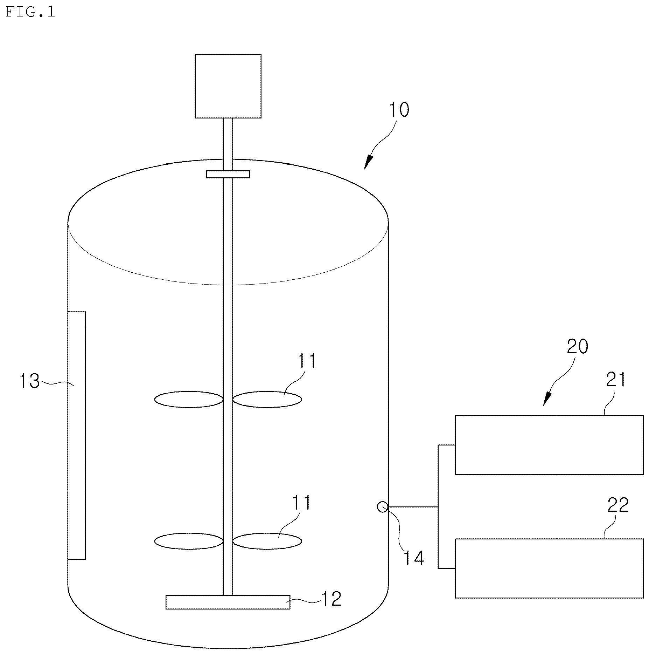

In order to solve this problem, as illustrated in FIG. 1, a conventional system for proliferating the cell culture can include a reactor 10 for accommodating the cell culture and a fluid medium, and an optical analyzer 20 which can continuously monitor the inside of the reactor 10.

More specifically, the reactor 10 can include a plurality of impellers 11 for mixing a stored cell suspension and a nutritional material, a sparger or an aeration 12 which is a device that is provided at the inner lower part to distribute gas in a liquid, a baffle 13 provided on an internal side wall, a port 14 which can collect a sample to the outside from the reactor 10 or can be inserted with a probe, etc. of an analyzer, and the like.

Further, the optical analyzer 20 can include a light source 21 and an analyzer 22 provided outside the reactor 10, and a probe (not illustrated), etc. which can be inserted into the reactor 10 through the port 14.

However, in the system for proliferating the cell culture, there are a large amount of bubbles generated by the impeller 11, the sparger or the aeration 12, etc. from the fluid medium and the cell suspension including a liquid and gas supplied and stored into the reactor 10.

Accordingly, the system for proliferating the cell culture has a problem in that when the probe is inserted into the reactor 10 and optical analysis by the optical analyzer 20 is performed, a concentration measurement error, etc. may occur by interference of the optical signal by the light scattering, etc., so the reliability of the analysis is reduced.

### SUMMARY

An exemplary embodiment has been made in an effort to provide a bubble separating apparatus for a spectrometer and a bioreactor including the same for continuously monitoring a cell culture inside the bioreactor without a need of collecting a sample to the outside from the bioreactor.

An exemplary embodiment has been made in an effort to provide a bubble separating apparatus for a spectrometer and a bioreactor including the same which separates bubbles which are present inside the bioreactor from being introduced into a detection region of the spectrometer to prevent interference of an optical signal due to the bubbles.

According to an exemplary embodiment of the present disclosure, a bubble separating apparatus for a spectrometer detachable to a spectrometer, which irradiates light through an irradiation unit provided at one end, and analyzes an analysis object based on characteristics of the light transmitting the analysis object in a detection region formed at a front side of the irradiation unit may be provided, which includes: a housing including an opening provided so that at least a part of the spectrometer is inserted at one end, and an irradiation hole provided so that the light irradiated from the irradiation unit passes at the other end; and a flange formed at the other end of the housing to be longer than the detection region in an irradiation direction of the light.

The housing may be provided in a hollow cylindrical shape in which one end at which the irradiation hole is provided is closed, and the opening is opened.

The housing may further include a cover opening and closing the opening.

The housing may be provided so as to interrupt introduction of a fluid into a space between an inner peripheral surface of the housing and an outer peripheral surface of the spectrometer into which at least a part of the spectrometer is inserted.

The flange may have an arc shape in which both ends are formed to be symmetric to be positioned at both sides of the irradiation hole based on a central portion provided on a virtual vertical line passing through a center of the irradiation hole in a radial direction, and heights of both ends may be the same as or higher than the center of the irradiation hole.

In the flange, both ends may be formed to be symmetric to be positioned at both sides of the irradiation hole based on a bending portion provided on a virtual vertical line passing through a center of the irradiation hole in a radial direction, and heights of both ends may be the same as or higher than the center of the irradiation hole.

At this time, the flange may further include a curved portion which is curved to the irradiation hole between the bending portion and both ends.

Meanwhile, according to an exemplary embodiment of the present disclosure, a bioreactor may be provided, which includes: a bubble separating apparatus for a spectrometer detachable to a spectrometer, which irradiates light through an irradiation unit provided at one end, and analyzes an analysis object based on characteristics of the light transmitting the analysis object in a detection region formed at a front side of the irradiation unit, in which the bubble separating apparatus for a spectrometer may include a housing including an opening provided so that at least a part of the spectrometer is inserted at one end, and an irradiation hole provided so that the light passes at the other end, and a flange formed at the other end of the housing to be longer than the detection region in an irradiation direction of the light, and the bioreactor may further include a gas inlet with a cylindrical shape, having an internal space for accommodating the analysis object and a fluid medium therein, and provided on the bottom surface of an internal space and supplying gas to the internal space, and a spectrometer insertion port provided at the center in an axial direction of the bioreactor, to which a spectrometer to which the bubble separating apparatus for a spectrometer is coupled is attached and detached.

Here, the detection region may be formed at the center in a diameter direction of the bioreactor.

In the bubble separating apparatus for a spectrometer and the bioreactor including the same according to the exemplary embodiments of the present disclosure, as the cell culture inside the bioreactor is continuously monitored without a need of collecting the sample to the outside of the bioreactor, the reliability of an analysis result can be enhanced.

In the bubble separating apparatus for a spectrometer and the bioreactor including the same according to the exemplary embodiments of the present disclosure, the interference of the optical signal is prevented by separating the bubbles which are present inside the bioreactor from being introduced into the detection region of the spectrometer, the reliability of the analysis result can be enhanced.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIG. 1 is a diagram illustrating a main configuration of a system for proliferating a cell culture in related art;
FIG. 2 is a cross-sectional view illustrating a state in which a bubble separating apparatus for a spectrometer is mounted on a spectrometer according to an exemplary embodiment of the present disclosure;
Each of FIGS. 3A, 3B, and 3C is a front view of the bubble separating apparatus for a spectrometer according to an exemplary embodiment of the present disclosure;
FIG. 4 is a diagram illustrating a main configuration of a bioreactor including the bubble separating apparatus for a spectrometer according to an exemplary embodiment of the present disclosure;
FIG. 5 is a graph showing a gas rising speed depending on a diameter in an internal space of the bioreactor according to the number of measurement times;
FIG. 6 is the graph showing the gas rising speed according to the number of measurement times in the internal space of the bioreactor of FIG. 4;
Each of FIGS. 7A, 7B, and 7C is a diagram illustrating a fluid flow of a detection region in a state in which a bubble separating apparatus for a spectrometer for each type according to an exemplary embodiment of the present disclosure is mounted on the bioreactor of FIG. 4; and
FIG. 8 is a graph showing a gas volume fraction in the detection region according to the bubble separating apparatus for a spectrometer for each type.

### DETAILED DESCRIPTION OF THE EMBODIMENT

Hereinafter, exemplary embodiments of the present disclosure will be described in detail with reference to the accompanying drawings. The following exemplary embodiment is to present the idea of the present disclosure to those skilled in the art to which the present disclosure pertains. The present disclosure is not limited to exemplary embodiments described presented herein and may be embodied in other forms. In the drawings, illustration of parts not related to the description to clarify the present disclosure is omitted, and the size of a component may be slightly exaggerated and expressed to help understanding.

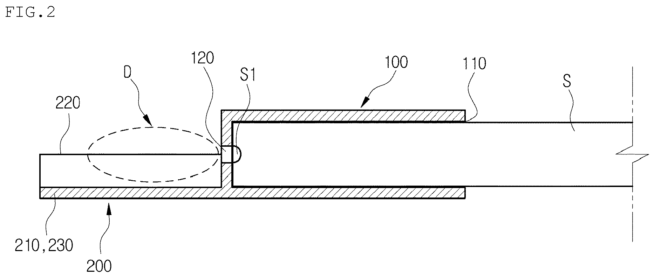

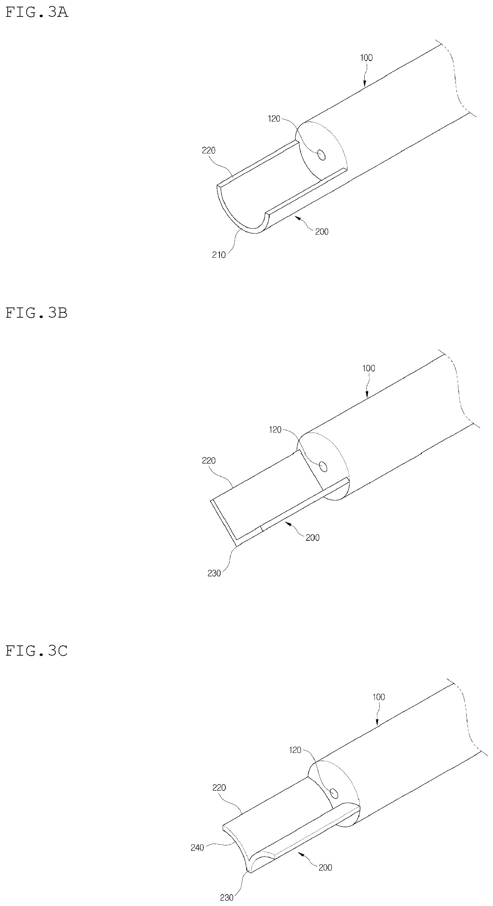

FIG. 2 is a cross-sectional view illustrating a state in which a bubble separating apparatus for a spectrometer is mounted on a spectrometer according to an exemplary embodiment of the present disclosure, and each of FIGS. 3A, 3B, and 3C is a front view of the bubble separating apparatus for a spectrometer according to an exemplary embodiment of the present disclosure.

First, a spectrometer S coupled to a bubble separating apparatus for a spectrometer according to an exemplary embodiment of the present disclosure has a generally cylindrical shape, and may irradiate light in an axial direction through an irradiation unit S1 provided at one end, and analyze an analysis object based on characteristics of light transmitting the analysis object in a detection region D formed at a front side of the irradiation unit S1. Here, the light irradiated through the irradiation unit S1 may include a multi-wavelength laser that emits light according to a plurality of different wavelengths in a near infrared region, and may be various lights such as light of a near infrared region (NIR), light of an infrared region (IR), light of an X-ray region (XRD), light of a radio wave region (NMR), and the like. Further, the spectrometer S may adopt a spectrometer including various known shapes and configurations.

Meanwhile, the bubble separating apparatus for a spectrometer according to an exemplary embodiment of the present disclosure includes a housing 100 and a flange 200.

The housing 100 includes an opening 110 provided so that at least a part of the spectrometer S is inserted at one end, and an irradiation hole 120 provided so that the light irradiated from the irradiation unit S1 passes at the other end. More specifically, in the housing 100, the spectrometer S may be coupled through the opening 110 so that the irradiation unit S1 is positioned in the irradiation hole 120 in a hollow cylindrical shape in which one end in which the irradiation hole 120 is provided is closed, and the opening 110 is opened. Further, the housing 100 may further include a cover (not illustrated) opening and closing the opening 110 in order to prevent foreign substances from being introduced into the inside while not being coupled to the spectrometer S. At this time, the cover may be provided to be opened and closed while being fastened to the opening 110 by a hinge, etc., or provided as a separate component so as to be separated from the housing 100.

Meanwhile, the housing 100 is not limited to the hollow cylindrical shape for coupling with the spectrometer S which may be provided in various shapes as described above, and is transformable according to the shape of the spectrometer S, of course. Further, as illustrated in FIG. 1, the housing 100 may be provided to interrupt introduction of a fluid into a space between an inner peripheral surface of the housing 100 and an outer peripheral surface of the spectrometer S formed by inserting at least a part of the spectrometer S.

The flange 200 may be formed at the other end of the housing 100 to be longer than a detection region D in an irradiation direction of light, and provided as a total of three types as illustrated in FIGS. 3A, 3B, and 3C.

More specifically, as illustrated in FIG. 3A, the flange 200 may have an arc shape in which both ends 220 are formed to be symmetric to be positioned at both sides of the irradiation hole 120 based on a central portion 210 provided on a virtual vertical line passing through a center of the irradiation hole 120 in a radial direction, and heights of both ends 220 may be the same as or slightly higher than the center of the irradiation hole 120. Hereinafter, the arc-shaped flange 200 is referred to as type A.

Meanwhile, as illustrated in FIG. 3B, in the flange 200, both ends 220 are formed to be symmetric to be positioned at both sides of the irradiation hole 120 based on a bending portion 230 provided on a virtual vertical line passing through a center of the irradiation hole 120 in a radial direction, and heights of both ends 220 may be the same as or slightly higher than the center of the irradiation hole 120. As a result, the flange 200 may be provided in a 'V' shape when viewed from the front. Hereinafter, the 'V'-shaped flange 200 is referred to as type B.

Further, as illustrated in FIG. 3C, the flange 200 may further include a curved portion 240 which is curved to the irradiation hole 120 between the bending portion 230 and both ends 220 in the B-type flange 200. Hereinafter, the flange 200 including the curved portion 240 is referred to as type C.

Further, as described above, the flanges 200 provided as three types are each integrally injected with the housing 100 and made of a synthetic resin material, and provided as a separate component to be fastened.

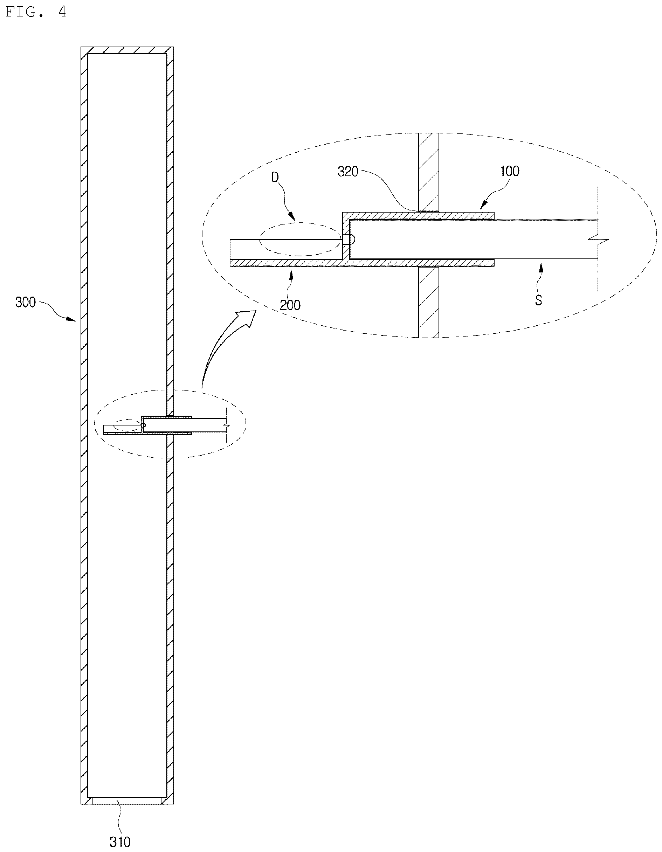

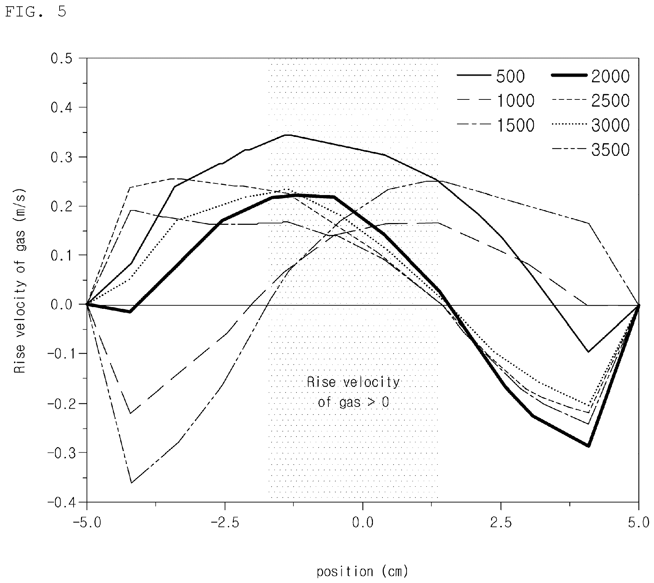

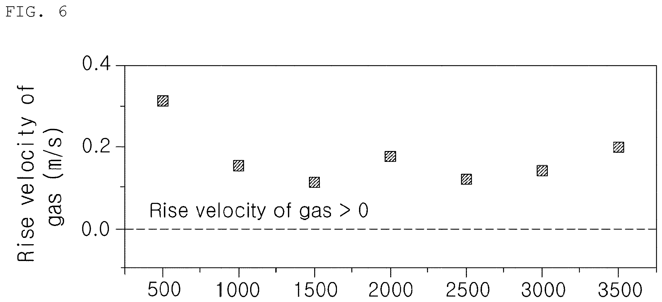

Meanwhile, FIG. 4 is a diagram illustrating a main configuration of a bioreactor including the bubble separating apparatus for a spectrometer according to an exemplary embodiment of the present disclosure, FIG. 5 is a graph showing a gas rising speed depending on a diameter in an internal space of the bioreactor according to the number of measurement times, and FIG. 6 is the graph showing the gas rising speed according to the number of measurement times in the internal space of the bioreactor.

By referring to FIGS. 4 to 6, a bioreactor 300 to which the bubble separating apparatus for a spectrometer according to an exemplary embodiment of the present disclosure configured as above is detachable has a cylindrical shape on the whole, and a space for accommodating an analysis object such as the cell culture, and a fluid medium is provided in the bioreactor 300. Further, the bioreactor 300 includes a gas inlet 310 provided on the bottom surface of an internal space and supplying gas to the internal space, and a spectrometer insertion port 320 provided at the center, to which the bubble separating apparatus for a spectrometer according to an exemplary embodiment of the present disclosure is attached and detached.

At this time, the gas inlet 310 includes a sparger or an aeration, and mixed gas of carbon dioxide (CO₂) and nitrogen (N₂) may be supplied to the analysis object such as the cell culture, and the fluid medium accommodated in the bioreactor 310 at an amount of 1.3 L/min to 13 L/min. Here, the type and a supply amount of gas supplied through the gas inlet 310 may depend on the analysis object, of course.

As illustrated in FIG. 4, the spectrometer insertion port 320 is provided at the center at an axial height of the bioreactor 300. Further, the spectrometer insertion port 320 may further include a cap (not illustrated) provided to prevent the fluid medium stored therein from being leaked while not being coupled to the bubble separating apparatus for a spectrometer according to an exemplary embodiment of the present disclosure.

As an example, referring to FIG. 5, it can be seen that in the bioreactor 300 configured as above with a height of 1.65 m and a diameter of 10 cm, the gas supplied through the gas inlet 310 shows an ascending flow at the center in a diameter direction of the bioreactor 300, and a descending flow toward the inner wall. More specifically, as illustrated in FIG. 6, according to an observation result according to the numbers of measurement times of 500, 1000, 1500, 2000, 2500, 3000, and 3500 times with respect to a continuous time, it can be seen that the gas supplied through the gas inlet 310 maintains an ascending flow of approximately 0.1 m/s to 0.35 m/s at all times at the center in the diameter direction of the bioreactor 300.

As a result, when the spectrometer S coupled to the bubble separating apparatus for a spectrometer according to an exemplary embodiment of the present disclosure is input into the bioreactor configured as above while being coupled to the bubble separating apparatus for a spectrometer, the spectrometer S is provided within a sufficient length so that the detection region D is positioned at the center of the diameter of the bioreactor 300.

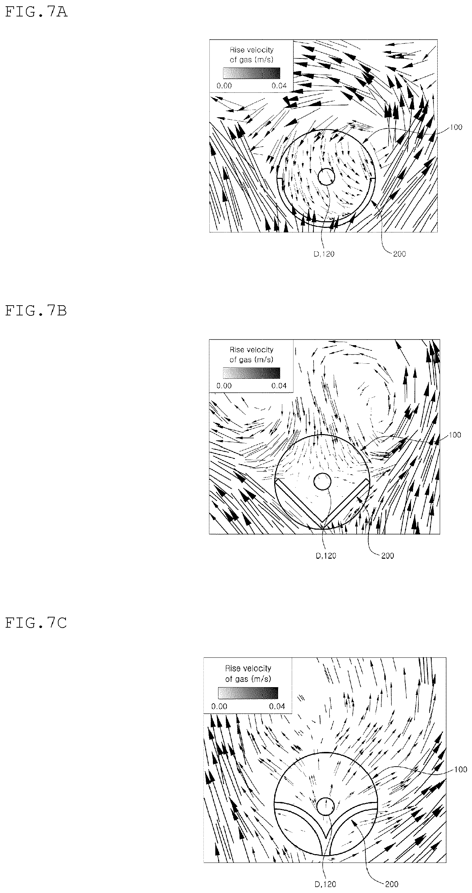

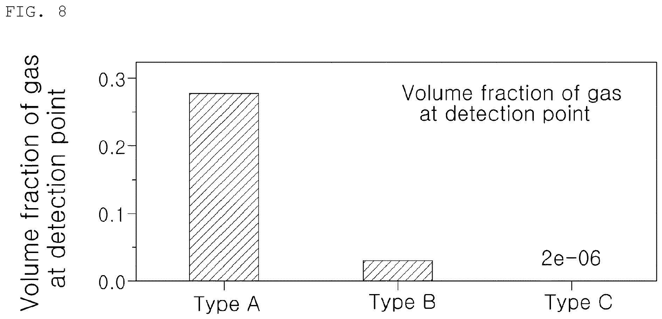

Each of FIGS. 7A, 7B, and 7C is a diagram illustrating a fluid flow of a detection region in a state in which a bubble separating apparatus for a spectrometer for each type according to an exemplary embodiment of the present disclosure is mounted on the bioreactor of FIG. 4, and FIG. 8 is a graph showing a gas volume fraction in the detection region according to the bubble separating apparatus for a spectrometer for each type.

Hereinafter, a flow of the gas supplied with the amount of 1.3 L/min to 13 L/min through the gas inlet 310 while the spectrometer S is input so that the detection region D is positioned at the center in the diameter direction of the bioreactor 300 while the bubble separating apparatus for a spectrometer according to an exemplary embodiment of the present disclosure configured as above is coupled to the spectrometer S will be described.

First, it can be seen that when the A-type flange 200 is applied, a gas volume fraction in the detection region D is shown as approximately 0.28 by a vortex phenomenon which occurs at both ends 220 as illustrated in FIGS. 7A and 8. However, compared to a state in which the bubble separating apparatus for a spectrometer according to an exemplary embodiment of the present disclosure is omitted, it can be seen that the bubbles in the detection region D are significantly reduced compared to bubbles in other regions.

Further, it can be seen that when the B-type flange 200 is applied, the gas volume fraction in the detection region D is shown as approximately 0.04 by the vortex phenomenon which occurs at both ends 220 as illustrated in FIGS. 7B and 8. That is, it can be seen that the bubble separating apparatus for a spectrometer including the B-type flange 200 has a better bubble blocking performance than the bubble separating apparatus for a spectrometer including the A-type flange 200.

Further, it can be seen that when the C-type flange 200 is applied, the gas volume fraction in the detection region D is close to 0 as illustrated in FIGS. 7C and 8. That is, it can be seen that the bubble separating apparatus for a spectrometer including the C-type flange 200 has a best bubble blocking performance among the three types of flanges 200.

As a result, in the bubble separating apparatus for a spectrometer and the bioreactor including the same according to the exemplary embodiments of the present disclosure, as the cell culture inside the bioreactor is continuously monitored without a need of collecting the sample to the outside of the bioreactor, the reliability of an analysis result may be enhanced, and the interference of the optical signal due to the bubbles is prevented by separating the bubbles from being introduced into the detection region of the analysis object, thereby significantly enhancing the reliability of the analysis result.

So far, the specific exemplary embodiments for the bubble separating apparatus for a spectrometer and the bioreactor including the same according to the present disclosure are described, but it is apparent that various modifications can be made within the limit without departing from the scope of the present disclosure.

Therefore, the scope of the present disclosure should not be limited and defined to the exemplary embodiments and should be defined by not only the appended claims but also equivalents to the appended claims.

That is, the exemplary embodiments described as above are exemplary in all aspects and should be understood as not being restrictive and the scope of the present disclosure is represented by claims to be described below rather than the detailed description, and it is to be interpreted that the meaning and scope of the claims and all the changes or modified forms derived from the equivalents thereof come within the scope of the present disclosure.

## Claims

1. A bubble separating apparatus for a spectrometer detachable to a spectrometer, which irradiates light through an irradiation unit provided at one end, and analyzes an analysis object based on characteristics of the light transmitting the analysis object in a detection region formed at a front side of the irradiation unit, the apparatus comprising:
a housing including an opening provided so that at least a part of the spectrometer is inserted at one end, and an irradiation hole provided so that the light passes at the other end; and
a flange formed at the other end of the housing to be longer than the detection region in an irradiation direction of the light.

2. The bubble separating apparatus for a spectrometer of claim 1, wherein the housing is provided in a hollow cylindrical shape in which one end at which the irradiation hole is provided is closed, and the opening is opened.

3. The bubble separating apparatus for a spectrometer of claim 2, wherein the housing further includes a cover opening and closing the opening.

4. The bubble separating apparatus for a spectrometer of claim 2, wherein the housing is provided so as to interrupt introduction of a fluid into a space between an inner peripheral surface of the housing and an outer peripheral surface of the spectrometer into which at least a part of the spectrometer is inserted.

5. The bubble separating apparatus for a spectrometer of claim 1, wherein the flange has an arc shape in which both ends are formed to be symmetric to be positioned at both sides of the irradiation hole based on a central portion provided on a virtual vertical line passing through a center of the irradiation hole in a radial direction, and heights of the both ends are the same as or higher than the center of the irradiation hole.

6. The bubble separating apparatus for a spectrometer of claim 1, wherein in the flange, both ends are formed to be symmetric to be positioned at both sides of the irradiation hole based on a bending portion provided on a virtual vertical line passing through a center of the irradiation hole in a radial direction, and heights of the both ends are the same as or higher than the center of the irradiation hole.

7. The bubble separating apparatus for a spectrometer of claim 6, wherein the flange further includes a curved portion which is curved to the irradiation hole between the bending portion and the both ends.

8. A bioreactor comprising:
a bubble separating apparatus for a spectrometer detachable to a spectrometer, which irradiates light through an irradiation unit provided at one end, and analyzes an analysis object based on characteristics of the light transmitting the analysis object in a detection region formed at a front side of the irradiation unit,
wherein the bubble separating apparatus for a spectrometer includes a housing including an opening provided so that at least a part of the spectrometer is inserted at one end, and an irradiation hole provided so that the light passes at the other end, and a flange formed at the other end of the housing to be longer than the detection region in an irradiation direction of the light, and
the bioreactor further includes a gas inlet with a cylindrical shape, having an internal space for accommodating the analysis object and a fluid medium therein, and provided on a bottom surface of the internal space and supplying gas to the internal space, and a spectrometer insertion port provided at a center in an axial direction of the bioreactor, to which the spectrometer to which the bubble separating apparatus for a spectrometer is coupled is attached and detached.

9. The bioreactor of claim 8, wherein the detection region is formed at a center in a diameter direction of the bioreactor.
